# EUROPEAN PATENT APPLICATION

(11) **EP 0 908 727 A1**
(43) Date of publication of application: **14.04.1999**
(21) Application number: 98307628.2
(22) Date of filing: 21.09.1998
(51) Int. Cl.: G01N 33/574, G01N 33/50

(54) **Synuclein-based assay and synuclein protein**

(30) Priority: 19.09.1997 GB 9719879
(71) Applicant: Neuropa Limited, Glasgow G11 6NU (GB); The University Court of The University of St Andrews, St Andrews, Fife KY16 9TS (GB)
(72) Inventor: The designation of the inventor has not yet been filed
(74) Representative: Ouzman, Beverley Nicola Claire

(57) **Abstract**

A novel member of the synuclein family has been identified and is termed persyn. The full amino acid sequence for the mouse and human isoforms are given in SEQ ID Nos 2 and 4, with the respective nucleotide coding sequences being given as SEQ ID Nos 1 and 3. The unusual expression pattern noted for persyn, in neurons and in skin, led to the realisation that synucleins affect intracellular intermediate filament structure. Thus, an assay for cells exhibiting abnormal intermediate filament structure is described, based upon evaluation of intracellular synuclein. The assay is special relevance to neurodegenerative disorders characterised by plaque deposition, to cancers (especially breast cancer) and to skin disorders. An assay for screening agents of potential therapeutic value is also described.

## Description

The present invention relates to an assay for detecting abnormalities in the intermediate filament structure of a cell as well as methods of diagnosing, gene therapy and methods of screening potential therapeutic agents for such abnormalities. A new protein, a novel synuclein, which led to the development of the assay is also described as well as polynucleotides (optionally in the form of genetic constructs) encoding the novel synuclein, and the use thereof in the assay.

The synucleins are a unique family of small intracellular proteins expressed in the nervous system and localised in synaptic terminals¹⁻⁶. α-Synuclein has been implicated in the pathogenesis of Alzheimer's disease and familial Parkinson's disease because it is present in senile plaques, binds amyloid Aβ peptide and is mutated in some families affected by Parkinson's disease⁷⁻¹⁰. However, the functions of synucleins remain elusive.

We have identified a novel member of the synuclein family termed persyn. This was cloned in experiments designed to isolate genes that are expressed in embryonic mouse sensory (E13 trigeminal ganglion) but not central (E13 forebrain) neurons. The longest of two independent, overlapping persyn clones was used to isolate full-length mouse and human cDNA clones. Figure 1 shows the high degree of homology between persyn and other synucleins. All of the EKTKEGV (SEQ ID No 16) repeats which are characteristic of the family¹⁻⁴ are conserved in persyn, and like most other synucleins persyn has a highly acidic C-terminal region. Persyn is most closely related to the rat synuclein-like protein¹⁵ and Torpedo synuclein¹, taking into account the evolutionary distance. However, substitutions of three lysines that are absolutely conserved in all other family members and an unusual stretch of five amino acids in another conserved region¹⁵ indicates that the rat synuclein-like protein is distinct from persyn and other synucleins.

The pattern of persyn mRNA expression differs from that of other synucleins. Briefly, persyn is expressed in the cytosol of sensory or motor neurons as opposed to α- and β-synucleins which are expressed in the synapse. The different pattern of expression reflects the way persyn was cloned. A single 0.8 kb transcript was expressed exclusively in neural tissues with the highest levels in sensory ganglia of both embryonic mice and newborn rats (Fig 2a, 2b). *In situ* hybridisation in whole mouse embryos and on cryosections revealed persyn expression in all sensory ganglia, motorneurons (Figs 2c, 2d, 2e) and postnatally in paravertebral sympathetic ganglia (data not shown). A detailed developmental study of persyn expression in the trigeminal ganglion revealed expression from the earliest stages of its formation with a marked increase in expression between E10 and E12, when the earliest axons are growing to their targets. This high level of expression was maintained in sensory neurons into adulthood (Northern data not shown, but see protein expression data, Fig 3e). Although no persyn mRNA and protein were detected in the cerebral cortex of postnatal and juvenile mice, persyn became clearly detectable in adult cortex and increased with age (Fig 3g). *In situ* hybridisation also showed that persyn mRNA was expressed in adult cortical neurons (see Fig 6 and Figure Legend). The level of persyn expression in the adult cerebral cortex was, however, still substantially lower than that in the spinal cord and sensory ganglia. In contrast to persyn, human α-and β-synucleins and rat, bovine and zebrafinch synucleins are predominantly expressed in the cerebral cortex and other forebrain structures³⁻⁶. Although the rat synuclein-like gene is predominantly expressed in sensory neurons, unlike persyn (Fig 2b), it is ubiquitously expressed in the central nervous system and other tissues of newborn rats¹⁵.

Western blotting using specific rabbit anti-persyn antibodies (raised against peptides illustrated in Fig 1) revealed a single protein in tissues that express persyn mRNA. This protein has a mobility corresponding to 19 kDa which is 6 kDa more than expected from the persyn sequence. Similar decreases in mobility have been reported for other synucleins^{4,16}. In development, the level of persyn expression in the trigeminal ganglion mirrored that of persyn mRNA (Fig 3e). Subcellular fractionation of mouse brain demonstrated that persyn is a cytosolic protein that is neither associated with cytoskeletal nor vesicular fractions (Fig 3f). This is similar to the intracellular distribution of rat synuclein and zebrafinch synelfin⁶. However, in contrast to other synucleins, which are predominantly presynaptic proteins⁵, persyn immunoreactivity was distributed diffusely throughout the cell body and axons of peripheral neurons both *in vivo* (Fig 3a, c) and *in vitro* (Fig 4).

We have found that persyn is able to self-aggregate to form homopolymers. Persyn also binds to amyloid β. Whilst we do not wish to be bound by theoretical considerations, the function of persyn appears to be to regulate the structure of intermediate filaments (eg. neurofilaments and cytokeratins) within a cell.

Persyn is nearly identical to a protein coded by an EST (BCSG1) isolated from a breast cancer cDNA library (41). Although BCSG1 mRNA cannot be detected in normal breast tissue and in benign breast tumours, its level dramatically increases in highly infiltrating malignant carcinomas (41). If the level of the encoded protein reflects the level of persyn/BCSG1 mRNA, it would be useful as a breast cancer progression marker.

We postulated (see Ref. 42) that the dual role of persyn in neurodegeneration and malignancy could involve common mechanisms. Changes in organisation of the cell cytoskeleton are among the most prominent characteristics of both processes. The involvement of persyn in regulating neurofilament network integrity raises the possibility that it may also affect the intermediate filament network in malignant breast epithelial cells. An important question is whether breast tumour progression correlates with a high level of expression of wild-type persyn or the presence of mutations in the persyn coding region is an obligatory characteristic of this process. Multiple differences (including three that resulted in amino acid substitutions) between BCSG1 isolated from tumour tissue and persyn isolated from normal brain tissue are consistent with the latter possibility. Such mutations could be somatic or germline and might reflect advanced tumour progression or predisposition of an individual to malignant progression of a tumour.

In one aspect, the present invention provides the novel protein persyn. We describe in detail the human and mouse isoforms of persyn, but the present invention is not limited to those isoforms alone. Amino acid sequence data for both the human and mouse isoforms of persyn are presented (SEQ ID Nos 2 and 4) and form a preferred embodiment.

In another aspect, the present invention provides a polynucleotide comprising a nucleotide sequence as set out in SEQ ID NO 1 or SEQ ID NO 3.

In a further aspect, the present invention provides a polynucleotide (for example a polynucleotide comprising a nucleotide sequence as set out in SEQ ID NO 1 or SEQ ID NO 3) encoding persyn.

The polynucleotides may be in any form (for example DNA or RNA, double or single stranded), but generally double stranded DNA is the most convenient. Likewise the polynucleotides according to the present invention may be present as part of a recombinant genetic construct, which itself may be included in a vector (for example an expression vector) or may be incorporated into a chromosome of a transgenic animal. Any vectors or transgenic animals comprising a polynucleotide as described above form a further aspect of the present invention.

In one embodiment the present invention provides a recombinant genetic expression system comprising a polynucleotide described above and able to express persyn.

Also included are recombinant systems which have been produced by knock-out of the gene naturally present, or by other similar techniques. In particular the present invention envisages targeting mutations (for example null mutations) within the gene in vivo.

The term "expression system" is used herein to refer to a genetic sequence which includes a protein-encoding region and is operably linked to all of the genetic signals necessary to achieve expression of the protein encoding region. Optionally, the expression system may also include a regulatory element, such as a promoter or enhancer, to increase transcription and/or translation of the protein-encoding region, or to provide control over expression. The regulatory element may be located upstream or downstream of the protein-encoding region, or may be located at an intron (non-coding portion) interrupting the protein encoding region. Alternatively the regulating protein may be expressed from a gene encoded on another plasmid (optionally stably integrated in the genome), acting in trans on a regulatory element situated upstream or downstream of a synuclein encoding sequence which may be present on another plasmid (optionally also stably integrated elsewhere in the genome). It is also possible for the sequence of the protein-encoding region itself to comprise a regulatory ability.

Additionally, the present invention comprises a vector containing such a recombinant expression system and host cells transfected with such a recombinant expression system (optionally in the form of a vector).

Suitable host cells will depend upon the vector and there is now a vast amount of information available to those skilled in the art on the selection of host cells. The invention encompasses microbial, animal and plant host cells. Preferred host cells include keratinocyte, neural progenitor, neuroblast and neural-like cell lines.

In particular one preferred embodiment is a stable cell line able to express persyn (preferably the mouse or human isoforms) under the influence of an inducible promoter system, especially the Teton inducible system (see Example 3).

In a further aspect, the present invention provides a transgenic mammalian animal, said animal having cells incorporating a recombinant expression system adapted to express persyn (preferably human or mouse persyn). Generally the recombinant expression system will be stably integrated into the genome of the transgenic animal and will thus be heritable so that the offspring of such a transgenic animal may themselves contain the trans gene and thus also be covered by the present invention. Suitable transgenic animals include (but are not limited to) mice, rats, rabbits, sheep, pigs, cattle and goats.

In a yet further aspect the present invention provides persyn produced by expression of a polynucleotide described above, preferably the genetic recombinant expression system of the present invention, desirably such persyn produced in a transgenic mammal.

In one embodiment, the construct is a cDNA construct designated hPERS-GEX encoding for human persyn or is a cDNA construct designated mPERS-BSII for mouse persyn. The constructs hPERS-GEX and mPERS-BSII have been deposited on 18 September 1997 at NCTC (Central Public Health Laboratory, London) under Accession Nos NCTC 13049 and NCTC 13050 respectively. hPERS-GEX is an expression vector whereas mPERS-BSII is a non-expressing vector.

Likewise transgenic mammals comprising the specific constructs listed above or modifications thereof are preferred.

Genetic engineering has been recognised as a powerful technique not only in research but also for commercial purposes. Thus, by using genetic engineering techniques (see Maniatis et al Molecular Cloning, a Laboratory Manual Cold Spring, Harbor Laboratory, Cold Spring Harbor, New York 1982 and "Principle of Genetic Engineering", Old and Primrose, 5th edition, 1994, both incorporated herein by reference) exogenous genetic material can be transferred to a host cell and the protein or polypeptide encoded by the exogenous genetic material may be replicated by and/or expressed within the host. For the purposes of simplicity genetic engineering is normally carried out with prokaryotic micro-organisms, for example bacteria such as E. coli, as host. However, use has also been made of eukaryotic organisms, in particular yeasts or algae, and in certain applications eukaryotic cell cultures may also be used.

Our experimental work has shown that over-expression of persyn in neurons leads to changes in the existing neurofilament network (see Example 2), believed to be degradation of the neurofilament proteins. These changes are suppressed by the addition of inhibitors of calcium dependent proteases which indicates that persyn, and the other synucleins, function by rendering certain structural proteins more susceptible to the proteases, for example by inducing a structural change. Similar affects in cytokeratins also occur in mature keratinocytes. The structural proteins so affected are herein designated "synuclein-sensitive".

Experimental overexpression of persyn in cultured sensory neurons results in the rapid decrease of immunoreactivity for all three neurofilament proteins. Other cytoskeletal components, for example actin, are not affected by persyn. Persyn displays distinctive patterns of expression in subsets of neurons and their axons in the developing and ageing nervous system and is concentrated in cortical axonal lesions seen in neurodegenerative conditions. We have found an accumulation of persyn in the ageing cortex. These findings indicate that persyn plays a role in modulating axonal architecture in the developing and mature nervous system and implicate persyn in the axonal pathology of degenerative conditions.

Additionally, we have detected substantial levels of persyn in skin; the only non-neural tissue noted for significant persyn expression. Specifically, persyn is expressed in the *Stratum granulosum* of the skin epidermis (this has a role in the later stages of skin development). Persyn overexpression has been observed in mature keratinocytes, indicating that the protein may be involved in dermatological conditions.

Interestingly, it has been proposed that α-synuclein functions as a molecular chaperon in the formation of Aβ-amyloid in the CNS of Alzheimer's disease patients⁷ and that mutated α-synuclein could have a similar function in the formation of Lewy bodies in familial Parkinson's disease²⁵. However, it could not be excluded that in certain situations synucleins themselves produce extra- or intracellular depositions because they are able to form homopolymers and amyloid-like structures *in vitro*^{8,9}. We have shown that recombinant persyn also aggregates in solution and binds Aβ-peptides with very similar binding characteristics to that of α-synuclein⁷ (Fig 5c, d). Another potentially important observation is that in both mouse and human persyn, amino acid 53 (labelled by an asterisk in Fig 1) is not alanine as in wild type α-synuclein, but threonine, as in the mutated α-synuclein of certain Parkinson's disease families¹⁰. It is possible that this alanine/threonine substitution causes changes in the secondary structure of α-synuclein that have functional consequences^{10,25}.

It has further been observed that the accumulation of intracellular or extracellular deposits in the form of plaques, tangles or inclusion bodies is a hallmark of many neurodegenerative diseases^{11,12}. These deposits are composed of one or more aggregated proteins and peptides, and it has been suggested that certain minor polypeptide components initiate aggregation of the main component^{13,14}. One of such minor component found in the plaques of Alzheimer's disease is NAC, an internal peptide or NACP or α-synuclein, a member of a family of small proteins with an unusual amino acid sequence and undefined functions¹⁻⁴. The ability of α-synuclein to self-aggregate and to bind and induce the aggregation of amyloid Aβ peptide⁷⁻⁹ are consistent with a potential role in promoting amyloid deposition in Alzheimer's disease. The topography of synaptic function impairment in Alzheimer's disease also correlates with the pattern of α-synuclein expression and its localisation in synaptic terminals³⁻⁶. Furthermore, synuclein has been implicated in at least one form of learning and memory in zebrafinch⁶. A mutation in the α-synuclein gene has also recently been reported to be directly associated with an early onset, autosomal-dominant form of Parkinson's disease¹⁰.

Consequently, following our observations on persyn, the role of the synucleins in certain disease states or disorders characterised by overdegradation or accumulation of synuclein-sensitive proteins became apparent.

In a further aspect, the present invention provides a method of diagnosis of, or determining the suspectibility to, disease states or disorders characterised by overdegradation or accumulation of synuclein-sensitive proteins, said method comprising:
a) obtaining an appropriate tissue sample from a patient;
b) analysing the presence or characteristics of a synuclein from said sample; and
c) comparing the results obtained to a known standard.

In one embodiment, the present invention provides a method of diagnosis of, or determining the suspectability to, disease states or disorders characterised by overdegradation or accumulation of synuclein-sensitive proteins, said method comprising:
a. obtaining an appropriate tissue sample from a patient;
b. determining the *ex vivo* level of a synuclein (for example persyn) in said sample; and
c. comparing that level to a known standard.

In another embodiment, the present invention provides a method of diagnosis of, or determining the susceptibility to, disease states or disorders characterised by overdegradation or accumulation of synuclein-sensitive proteins, said method comprising:
a. obtaining an appropriate tissue sample from a patient;
b. isolating a synuclein (for example persyn) or a polynucleotide encoding therefor, from said sample;
c. comparing said synuclein or said polynucleotide to a known standard and determining whether there are any relevant differences (for example differences in sequence or physical characteristics) therein.

The overdegradation or accumulation of synuclein-sensitive proteins may be accompanied by an alteration in intracellular intermediate filament structure.

The methods described above will be of particular utility in the diagnosis of conditions in which increased intracellular levels (especially overexpression) of a synuclein, preferably persyn, are a significant factor.

Likewise, the identification of mutations within α-synuclein are of particular interest.

In one embodiment the methods described above may be directed towards the diagnosis of neurodegenerative disease.

In a further embodiment the methods described above may be directed towards the diagnosis of highly metastatic cancers.

In particular, the assay described above is of relevance with regard to screening, diagnosis or monitoring cancers (especially breast cancer and skin cancer), of neurodegenerative disorders (especially disorders where abnormal deposition of protein/peptides is typically observed) and of skin disorders (especially tumours of epithelial origin). With regard to the use of the method for cancer, the level and effect of abnormal synuclein presence could be useful as an indicator of tumour tissue, as well as indicating the progression and metastasis of the malignancy.

The "synuclein" which is the subject of the assays mentioned above may be persyn or any related protein, such as α-synuclein (especially human or mouse) or β-synuclein (especially human or mouse). The full nucleotide sequence encoding mouse α-synuclein is available under Accession No AF044672, Nucleotide Identification No g282929. With regard to persyn itself, the mouse or human isoforms are preferred, but other naturally occurring isoforms (e.g. rat) may be acceptable. Our preliminary studies indicate that overexpression of α-synuclein demonstrates similar disruptions of neurofilament proteins within cultured cells. In methods requiring a DNA probe, it may not be necessary to use the whole of the genetic sequence of the isoform of synuclein selected, provided that the specificity of the probe is maintained. Likewise, modifications to the sequence (especially if the probe is manufactured synthetically) may be tolerated, provided that the specificity remains acceptable.

The invention also encompasses a method of evaluating the genomic sequence of endogenous synuclein (for example persyn or α-synuclein) to establish whether any mutation or abnormality is present, and if so optionally to identify that mutation or abnormality. The genomic sequence of the endogenous protein will be determined by conventional techniques and the sequence obtained compared to the known sequence, for example the sequence set out in SEQ ID No 1. This method can be used in diagnosis, and also in prognosis of diseases or disorders, especially in human medicine. For example the method may have utility in gamete or fetal screening.

In another aspect, the invention also provides a method of combatting intermediate filament damage in cells due to abnormal levels of synuclein (for example, keratinocytes or neural tissue), the method comprising identifying the cells so damaged and modifying the biologically effective amount of synuclein (for example persyn) in the cells. Usually, it will be desirable to reduce the amount of endogenous functional synuclein (for example persyn), but in certain instances increasing the amount of endogenous functional synuclein (for example persyn) may be advantageous.

The biologically effective amount of synuclein (for example persyn) may be modified by affecting its expression; ie by affecting the amount of synuclein (for example persyn) being generated from nucleic acid eg by homologous recombination of defective versions of persyn; alternatively, the synuclein (for example persyn) mRNA can be neutralised by providing antisense RNA; alternatively, a recombinant expression system can be used to produce an additional amount or an alternative form of synuclein (for example persyn). Ribozymes specific to persyn or other synucleins may also be of utility in this regard. In a different approach the function of the synuclein (for example persyn) protein can be modified by therapeutic agents which affect its function.

By "combatting" we mean amelioration of existing damage and/or prevention of, or protection against, damage before it may occur.

Persyn according to the invention and the other proteins of the synuclein family are also of utility in a method of screening for potential therapeutic agents effective in modifying the biological activity of a synuclein, especially with regard to the effect upon the sensitivity of certain structural proteins to Ca²⁺ dependent proteases (the synuclein-sensitive proteins).

According to a yet further aspect, the present invention provides a method of screening for a therapeutic agent able to combat the biological activity of a synuclein (eg persyn), said method comprising:
a. obtaining a system capable of demonstrating the biological activity of the synuclein (for example primary cell cultures or cell lines adapted to express synuclein-sensitive proteins, such as a mature keratinocyte, neural progenitor, neuroblast or neural-like cell line transfected with a persyn expression system or micro-injected with persyn);
b. exposing said system to a putative therapeutic agent; and
c. determining whether said agent affects the biological activity of the synuclein.

For convenience step c. may involve immunofluorescent staining, as described in detail in Example 2.

In respect of all of the methods described above (including the methods of diagnosis or prognosis, the method of evaluating the genomic sequence, the method of combatting intermediate filament damage and the method of screening for a therapeutic agent) the synuclein in question is preferably expressed from a stable cell line. Desirably the cell line is a keratinocyte, neural progenitor, neuroblast or neural-like cell line. Desirably synuclein expression is under the influence of an inducible promoter system, preferably the Teton inducible system (see Example 3).

With regard to all of the assays and methods described above, the synuclein may be persyn (as defined in SEQ ID Nos 2 or 4). Additionally, functional equivalents of persyn, modifications of persyn and portions of persyn or persyn functional equivalents or modifications may also be suitable (generally such portions will be those unique to persyn).

The term "functional equivalent" refers to any derivative which is functionally substantially similar to the reference sequence or protein. In particular the term "functional equivalent" includes derivatives in which nucleotide base(s) and/or amino acid(s) have been added, deleted or replaced without a significantly adverse effect on biological function.

The term "modification" includes derivatives substantially similar to the reference sequence or protein. In particular the term "modification" includes derivatives in which nucleotide(s) and/or amino acid(s) have been added, deleted or replaced affecting biological-function in a desired manner. Modifications covered include domain rearrangement proteins, fusion or chimeric proteins (having persyn and non-persyn portions) and the like.

Embodiments of the present invention will now be described by way of example, and with reference to the accompanying drawings and Sequence Listings in which:
- SEQ ID No 1 :: nucleotide sequence encoding human persyn
- SEQ ID No 2 :: amino acid sequence for human persyn
- SEQ ID No 3 :: nucleotide sequence encoding mouse persyn
- SEQ ID No 4 :: amino acid sequence for mouse persyn
- SEQ ID No 5 :: amino acid sequence for torpedo californica synuclein (Fig. 1)
- SEQ ID No 6 :: amino acid sequence for rat synuclein 1 (Fig. 1)
- SEQ ID No 7 :: amino acid sequence for rat synuclein 2 (Fig. 1)
- SEQ ID No 8 :: amino acid sequence for rat synuclein 3 (Fig. 1)
- SEQ ID No 9 :: amino acid sequence for human α-synuclein (Fig. 1)
- SEQ ID No 10 :: amino acid sequence for human β-synuclein (Fig. 1)
- SEQ ID No 11 :: amino acid sequence for bovine synuclein (Fig. 1)
- SEQ ID No 12 :: amino acid sequence for zebrafinch synuclein (Fig. 1)
- SEQ ID No 13 :: amino acid sequence for rat synuclein-like (Fig. 1)
- SEQ ID No 14 :: sense oligonucleotide probe (Example 3)
- SEQ ID No 15 :: anti-sense oligonucleotide probe (Example 3)
- SEQ ID No 16 :: amino acid motif characteristic of synucleins (see page 2).

Fig 1 shows homologies between mouse persyn and other members of synuclein family. Amino acids which are not identical in all or in all but one members of the family are shown in bold. Gaps (-) were introduced for better alignment. The peptides used as antigens for antibody production are boxed. The asterix marks amino acid 53 which in both human and mouse persyn is not alanine as in wild type α-synuclein, but threonine, as in the mutated α-synuclein of certain Parkinson's disease families¹⁰.

Sequence accession numbers: Torpedo californica synuclein - P37379; rat synucleins 1, 2, 3 - products of alternative splicing of the same gene - S73007, S73008, S73009; human α-synuclein (synuclein 1/NACP) - L36674; human β-synuclein (synuclein 2) - S69965; bovine synuclein - P33567; zebrafinch synelfin - L33860; rat synuclein-like - X86789.

Fig 2 shows expression of persyn mRNA in mouse and rat tissues: (a,b) Northern hybridisation of 5-20 µg of total RNA with a nick-translated persyn probe. RNA was isolated from E13 mouse embryonic tissues (a) and newborn rat tissues (b). After stripping off the probe, the same filter was hybridised with a nick-translated cDNA fragment encoding the mouse L27 ribosomal protein to provide an indication of the amount of total RNA from each tissue present on the filter. TG - trigeminal ganglia; DRG - dorsal root ganglia. (c-e), in situ hybridisation of embryonic mouse tissues with a DIG-labelled antisense cRNA persyn probe. (c), whole mount in situ hybridisation of E11 mouse embryo. TG, trigeminal ganglion; VG, vestibular ganglion; GG, geniculate ganglion; JG, jugular ganglion; NG, nodose ganglion; DRG, dorsal root ganglia; MN, motoneurons in ventral horns of the spinal cord. (d), transverse sections at the level of the forelimb buds of E10, E11 and E12 embryos. VSC, motoneurons in ventral horns of the spinal cord. Bar = 1 mm. (e), a fragment of coronal section of E11 mouse embryo head. TG, trigeminal ganglion; HB, hindbrain; OMN, oculomotor nucleus. Bar = 0.2 mm.

Fig 3 (a-d) shows immunohistochemical localisation of persyn protein in mouse neuronal cell bodies and processes: Detection of persyn protein in trigeminal nerve (a) and trigeminal ganglia neurons (c) on coronal section of E11 mouse embryo head using the anti-mouse persyn antibody and FITC-conjugated anti-rabbit immunoglobulins. (b, d), the same section stained with anti-neurofilament-M monoclonal antibody and TRITC-conjugated anti-mouse immunoglobulins. (e - g), Western blotting/ECL detection of persyn protein using the anti-mouse persyn antibody. (e), developmental time course of persyn expression. Equal amounts (15 µg) of total protein from embryonic (E) or newborn (P0) mouse trigeminal ganglia were analysed. (f), persyn in subcellular fractions from mouse brain. 20 µg of total proteins from subcellular fractions (see Examples) were analysed. (g), persyn expression in postnatal day 3 (P3), 7 week (7W) and 7 month (7M) spinal cord and cerebral cortex.

Fig 4 shows cytoskeletal proteins in neurons overexpressing persyn. Matching photomicrographs of cultures of postnatal sensory neurons double stained for persyn (FITC) and cytoskeletal proteins (TRITC) 72 hours after microinjection of persyn expression plasmid. Each micrograph contains injected and non-injected neurons. Note the decreased NF-M staining of neurons injected with the persyn expression plasmid compared with the uninjected neuron (similar results were obtained for other neurofilament proteins, see Fig 7). Incubation of injected neurons with calpeptin, an inhibitor of calcium-dependent proteases, suppressed the effect of persyn overexpression on neurofilament staining. Overexpression of persyn did not affect staining for tubulin and peripherin.

Fig 5 shows photomicrographs of cortical sections of dementia with Lewy bodies stained with anti-human persyn antibodies showing dot-like structures in cortical white matter (a) and absence of staining of cortical Lewy bodies (b). Bar charts showing binding of persyn to filter-immobilised Aβ peptides (c, d). 0.5 nmol of Ab 1-40, 1-28 or 25-35 peptides were spotted on nitrocellulose membrane filters and incubated in a 2 µM persyn solution at 37°C for 16 hours. The means and standard errors of three experiments are shown (c). 2 µM persyn was preincubated with 0, 10, or 50 µM of Ab 25-35 at 37°C for 16 hours after which it was incubated with filters on which 0.5 nmol of Ab 1-40 peptide was spotted. The means and standard errors of three experiments are shown (d).

Fig 6 shows persyn mRNA expression in adult mouse cortical neurons. In situ hybridisation of a cryosection of adult mouse brain with DIG-labelled persyn cRNA probe. A fragment of frontal cerebral cortex is shown.

Fig 7 shows a decrease of neurofilament immunostaining in cultured sensory neurons microinjected with persyn expression plasmid. An example of trigeminal neurons stained with an antibody specific to NF-M 24 hours after injection (a, c) and nodose neurons stained with an antibody specific to NF-H 72 hours after injection (b, d). Both normal (a, b) and persyn-overexpressing (c, d) neurons are present on each pair of microphotographs as visualised by the immunofluorescence staining observed. The figures demonstrate that red (c, d) and green (a, b) cells are in two mutually exclusive populations. (The yellow signals in cell bodies of "normal" neurons in upper panels (white arrows) are due to bleed-through of strong rhodamine fluorescence into the fluorescein channel). Also, note that although some residual neurofilament staining remains in the cell bodies of neurons that overexpressed persyn, the processes of these cells are virtually NF-negative. This creates completely nonoverlapping patterns of process staining in rhodamine and fluorescein channels.

Fig 8 shows ectopic expression of persyn in 3T3 fibroblasts does not influence vimentin immunostaining. Fibroblasts were transfected with a persyn expression plasmid and immunostained with rabbit polyclonal anti-persyn and mouse monoclonal anti-vimentin antibodies.

Fig 9 shows helical wheel displays of the fragments that possess intermediate filament-disruptive activity.

Fig 10 Persyn expression in stratum granulosum of skin epidermis. (a), in situ hybridisation of a cryosection with a DIG-labelled antisense cRNA *persyn* probe. A fragment of the mouse thick skin (transverse section of the footpad) is shown. (b,c), adjacent sections were used for immunohistochemical detection of persyn protein with affinity purified SK23 antibody and HRP-conjugated anti-rabbit immunoglobulins. Scales bars=200 µm for panels a and b; 50 µm for panel c.

Fig 11 Neuro2a (neuroblastoma) cells transiently transfected with a Persyn expression vector (pIRES-EGFP-Persyn). Fig A illustrates cells expressing Persyn as indicated by the concomitant expression of EGFP. Fig B illustrates cells stained for the neurofilament (NF-H) protein (Texas Red-X), 48 hours post transfection with the pIRES-EGFP-Persyn expression vector. Fig C illustrates the superimposition of images A and B. Note the dramatically decreased staining corresponding to neurofilament-H withing cells transfected with the pIRES-EGFP-Persyn expression/reporter plasmid, compared with cells which had not been transfected with pIRES-EGFP-Persyn.

### Example 1 : Cloning persyn and antibody binding

### MATERIALS AND METHODS

### Molecular cloning and hybridisation

A subtractive hybridisation technique³⁰ was used to isolate cDNA clones of mRNAs that are enriched in embryonic mouse trigeminal ganglia relative to E13 mouse forebrain. Among the clones isolated from the resulting subtracted cDNA library were two independent overlapping clones that represented persyn mRNA. Full-length cDNAs were isolated from an E13 mouse trigeminal cDNA library constructed in the "lambola" ZAPII vector and a cDNA library from juvenile human brainstem (Stratagene).

RNA extraction, preparation of hybridisation probes, Northern, in situ and Southern hybridisations and mouse E13 trigeminal ganglia cDNA library construction and screening were performed as described³¹.

### Production of recombinant Persyn and binding assays

The *Nco*I-*Xho*I fragment of the persyn cDNA was cloned in-frame into the *Sma*I site of the pGEX-4T-1 vector (Pharmacia). GST-fused persyn was isolated by affinity chromatography on glutathione Sepharose 4B (Pharmacia), digested with human thrombin, GST was re-absorbed on glutathione Sepharose 4B and the persyn was further purified by FPLC chromatography (Mono Q column, Pharmacia). In vitro binding of recombinant persyn to Aβ 1-40, 1-28 and 25-35 peptides (Bachem Bioscience) immobilised on membrane filters was carried out as described for recombinant α-synuclein/NACP⁷.

### Results

Full length mouse and human cDNA clones were obtained; the persyn mouse and human cDNA constructs mPERS-BSII and hPERS-GEX have been deposited at NCTC under Deposit Nos 13050 and 13049 respectively.

### Anti-persyn antibodies

Rabbits were immunised with the 15-mer C-terminal peptide of mouse persyn and an internal peptide of human persyn (amino acids 80-95) each conjugated with keyhole limpet haemocyanin (Calbiochem) activated by MBS (Sigma)³². Monospecific antibodies were purified from the respective antisera by affinity chromatography using the antigen bound to NHS-activated columns (Supelco). The anti-mouse and anti-human persyn monospecific antibodies were used at dilutions of 1:500 and 1:200, respectively, for Western blot/ECL detection of persyn in total cell protein samples as described previously³³. Rainbow markers from Amersham were used as protein size standards.

### Results

Western blotting using specific rabbit anti-persyn antibodies (raised against peptides illustrated in Fig 1) revealed a single protein in tissues that express persyn mRNA. This protein has a mobility corresponding to 19 kDa which is 6 kDa more than expected from the persyn sequence.

### Example 2 : Overexpression of persyn in neurons and immunohistochemistry

### MATERIALS AND METHODS

### Subcellular fractionation

Fractionation of homogenates of the hindbrains of adult mice was carried out as described previously^{34,35}. 20 µg of total protein from each fraction of the 1,000g supernatant (cyt), 20,000g supernatant (pmt), 20,000g supernatant after washing of 20,000g pellet (pl), mitochondrial (mt) and synaptosomal (syn) fractions of sucrose gradient were analysed by Western blotting/ECL detection.

### Immunohistochemistry

15 µm cryosections were fixed in acetone at -20°C for 10 minutes and were dried and blocked with 10% goat serum in TBT (20 mM TrisHCl pH7.5; 150 mM NaCl; 0.1% Triton X-100) followed by incubation with the anti-mouse persyn antibody (1:50) at 4°C for 16 hours in 1% goat serum/TBT. Sections were washed with 1% goat serum/TBT. A similar procedure was used for immunostaining neurons in culture after fixing the cells with acetone/methanol (1:1) and washing with TBT before blocking. The following monoclonal antibodies were used for detecting cytoskeletal proteins: clone 34 (Affinity) and clone NR4 (Boehringer Mannheim) for NF-L, clone NN18 (Sigma) for NF-M, clone RT97 (Boehringer Mannheim) and SMI312 (Sternberger Monoclonals) for NF-H, MAB1527 (Chemicon) for peripherin, clone V9 (Sigma) for vimentin, clone TUB2.1 (Sigma) for β-tubulin. TRITC-conjugated philoidine was used to stain actin microfilaments. TRITC-conjugated anti-rabbit and FITC-conjugated anti-mouse secondary antibodies (Jackson's Laboratories) were used in these experiments.

Staining of formalin-fixed paraffin sections of human brain samples with anti-human persyn antibody (1:20) was carried out as described earlier^{28,29}.

### Dissociated neuronal culture and microinjections

Mouse P1 sensory neurons were maintained in culture in the presence of either 2 ng/ml of NGF (trigeminal ganglia neurons) or 2 ng/ml of BDNF (nodose ganglia neurons) for 5 days as described³⁶ before microinjection with expression plasmid (mouse persyn cDNA in pMEX vector)³⁷. In some experiments calpeptin or leupeptin (10 mM) were added to the culture medium after microinjections.

### Results : Overexpression of persyn in neurons

To investigate the consequences of persyn overexpression in neurons, we microinjected cultured sensory neurons with a persyn expression plasmid. Injected neurons showed intense persyn immunofluorescence whereas uninjected neurons had a much lower level of staining. A dramatic decrease of triplet neurofilament protein staining in neurons overexpressing persyn was observed compared with uninjected neurons in the same culture dish. This marked decrease in immunostaining was revealed with several monoclonal antibodies against each of the three neurofilament proteins: NF-L, NF-M and NF-H (Fig 4, and Fig 7 - see also Figure Legends). Similar results were obtained using neonatal trigeminal and nodose neurons. The decrease in neurofilament staining was evident 24 hours after injection and was more prominent after 48 and 72 hours. The intensity of neurofilament staining in neurons overexpressing persyn was lower than at the time of injection, indicating that persyn, at least in part, induces changes in the pre-existing neurofilament network. No changes in immunostaining for microfilaments (actin), microtubules (tubulin) or another intermediate filament (peripherin) were observed in injected neurons (Fig 4). Also, ectopic expression of persyn in fibroblasts did not change the pattern of staining for vimentin, the major intermediate filament of these cells (see Fig 8 and also Figure Legends).

Distortions of the neurofilament network are not only common in neurodegenerative diseases but are also observed following traumatic injury, ischemia and exposure to neurotoxic agents. These acute insults frequently cause a rapid decrease in neurofilament immunostaining that is believed to result from the degradation of neurofilament proteins by calcium-dependent proteases¹⁷⁻²⁰. Inhibitors of these proteases prevent this decrease and associated neuronal damage²⁰⁻²². Here we show that calpeptin and leupeptin, inhibitors of calpain and other calcium-dependent proteases, suppress the effect of persyn overexpression on neurofilament proteins (Fig 4). This suggests that overexpression of persyn in neurons leads to increased degradation of neurofilament proteins by calcium-dependent proteases. This cannot be due to a general increase in protease activity because other cytoskeletal proteins that are also substrates for these proteases are not affected. Although we do not wish to be bound by hypotheses, one explanation is that persyn changes structural aspects of neurofilaments, making them more susceptible to the action of proteases. Interestingly, there are sequence similarities between the synuclein EKTKEGV repeats and a region of the helical rod domains of neurofilament proteins that is important for neurofilament network assembly. Neurofilament mutants lacking this region fail to integrate into neurofilamentous arrays in transfected cells and expression of peptides corresponding to this region leads to the disintegration of pre-existing filament arrays²³. Similar intermediate filament-disruptive effects have been demonstrated for peptides corresponding to the same regions of the helical rod domains of vimentin and keratins *in vivo* and *in vitro*²⁴. Helical wheel displays of these regions are strikingly similar to helical wheel displays of synuclein 11-mer repeats (see Fig 8 and also Figure Legend). This raises the possibility that interactions involving such amphipathic α-helices is the molecular basis of the observed effects of intermediate filament fragments and persyn protein. However, our immunostaining and cell fractionation results (Fig 3) as well as co-immunoprecipitation and in vitro binding experiments (not shown) clearly demonstrate that persyn is not normally a component of neurofilament arrays and does not bind tightly to neurofilament proteins. It is therefore possible that persyn acts as a molecular chaperon that regulates neurofilament network integrity.

### Results : Immunohistochemistry

The structural and biochemical features of persyn together with its expression in ageing mouse cerebral cortex indicate that it might be involved in physiological and pathological changes in the ageing human brain. To investigate this possibility we used immunohistochemistry to study persyn expression in post-mortem brain samples from patients that suffered from neurodegenerative diseases and control samples from individuals of the same age group. In both normal and diseased cerebral cortex, persyn exhibited patchy immunoreactivity that is characteristic of synaptic marker staining, a pattern that has also been observed for synucleins⁵. Unlike peripheral axons, which are intensively stained for persyn, cortical axons were not obviously stained. However, persyn-immunoreactive dot-like structures were observed in the white matter of diseased cortex (Fig 5b). These structures correspond to ubiquitin-immunoreactive lysosomal dense bodies within axons that accompany normal ageing but are increased in neurodegenerative disease^{26,27}. Similar dot-like structures have also been noted in association with prion diseases^{28,29}. In the limited number of cases of Alzheimer's disease and dementia with Lewy bodies studied, persyn immunoreactivity was not observed in cortical tangles, amyloid plaques or Lewy bodies (Fig 5a). These findings may implicate persyn in axonal pathology. Thus, it will be important to study further persyn's involvement in neurodegenerative diseases, including peripheral neuropathies because of the high level of persyn expression in the PNS.

In conclusion, we have identified a novel member of synuclein family of proteins with distinct patterns of expression in the developing and ageing nervous system. In demonstrating that persyn influences neurofilament network integrity, we have assigned a potential function to the synuclein family, a function that may have important implications for understanding the pathophysiology of neurodegenerative diseases.

### Example 3

### Construction of synculein vector systems Persyn cloning into pTRE

This example describes the cloning of persyn into pTRE and the generation of a plasmid system permitting the generation of stable cell lines expressing a synuclein (persyn) under the inductive control of tetracycline or a tetracycline analogue. Thus, the resulting constructs are to be used in conjunction with the pTet^{on} (CLONTECH) plasmid in accordance with manufacturer's instructions.

Sense oligonucleotide: 5′ GGA ATT CCA TCC TTG CAA ACA
CCA TGG 3′ (SEQ ID No 14).

Antisense oligonucleotide: 5′ CGG GAT CCC TAG TCT TCT
CCA CTC TTG GC 3′ (SEQ ID No 15).

### PCR

30 pMoles of each oligonucleotide (defined above) were used to PCR amplify the cDNA fragment encoding Persyn from within pMEX-Persyn expression plasmid (Buchman et al,Nature Neuroscience, Vol 1, No. 2, June 1998). The pMEX-Persyn template DNA was in the form of a scraping that had been taken from a -20°C stock (Glycerol; Luria broth; 1:1) of the plasmid. Ths scraping of cells was placed in 100µl of dH₂O and incubated at 100°C for 10 minutes to lyse the cell walls of the bacterial host and release the plasmid DNA into solution. 1µl of the cell lysate was utilised per 100 µl reaction.

PCRs were performed in the presence of: 1 x PCR buffer (Boehringer Mannheim, High Fidelity PCR system); 1.5 mM MgCl₂; 200 µM dNTP; 2.6 U High Fidelity PCR system enzyme (Boehringer Mannheim).

PCR conditions were as follows:

| | |
|---|---|
| 94°C 2 minutes | |
| 94°C 15 seconds | * |
| 57°C 30 seconds | * |
| 72°C 1 minute | * 20 cycles |
| 72°C 10 minutes | |

| | |
|---|---|
| * indicates sequential time periods at set temperatures that when completed comprise a single cycle. Twenty cycles were performed per PCR. | |

PCR products were extracted with an equal volume of Chloroform. To each reaction 1/10 volume of 3M sodium acetate (pH 5.2) and 2 volumes 100% ethanol were added. They were subsequently placed at -20°C for 60 minutes and then centrifuged at 14,000 rpm for 20 minutes and the supernatant aspirated. 500 µl of ethanol (70%) was added to each tube. The tubes were centrifuged at 15,000 rpm for 2 minutes and the supernatant aspirated. The pellet was air dried for approximately 10 minutes and resuspended in 90 µl of dH₂O.

The PCR products were then cleaved by incubation in the presence of 20 U *Eco*RI and 20 U *Bam*HI (Gibco BRL™) and 1 x reaction buffer 3.

The sample was electrophoresed in a 1% ordinary agarose gel. A DNA fragment, corresponding to the cDNA fragment encoding Persyn, was isolated from the agarose using QIAquick (QIAgen™) in exact accordance with the manufacturer's instructions.

pTRE (Clontech) was cleaved in the presence of *Eco*RI and *Bam*HI (Gibco BRL™) as above. To each reaction 1/10 volume of 3M sodium acetate (pH 5.2) and 2 volumes 100% ethanol were added. They were subsequently placed at -20°C for 60 minutes and then centrifuged at 14,000 rpm for 20 minutes and the supernatant aspirated. 500 µl of ethanol (70%) were added to each tube. The tubes wre centrifuged at 15,000 rpm for 2 minutes and the supernatant aspirated. The pellet was air dried for approximately 10 minutes and resuspended in 10 µl dH₂O.

Each remaining sample was then electrophoresed in a 1% ordinary agarose gel. The PCR amplified DNA fragment, corresponding to the cDNA fragment encoding Persyn, was isolated from the agarose using QIAquick (QIAgen™) in exact accordance with the manufacturer's instructions.

The cDNA fragment encoding Persyn was ligated to the linearised pTRE vector in a molar end ratio of 3:1 (insert:vector), by incubation (room temperature, 18 hours) in the presence of 0.5 U T4 DNA ligase and 1 x ligation buffer (Promega). Ligated DNAs were transformed into a transformation competent Top 10 *E.coli* (Invitrogen®) bacterial host. DNAs from resulting transformants were examined using *Taq* cycle sequencing to verify the presence of the cDNA fragment encoding mouse Persyn, and to confirm that the known open reading frame had been conserved.

### Example 4

### Construction of synuclein expression vector systems

### Constitutive

1 µg of the pIRES-EGFP Vector (CLONTECH) was cleaved by incubation (37°C, 60 minutes) in the presence of 50 U (Weiss units) *Bst* XI and 1 x reaction buffer D (Boehringer Mannheim). The vector (pTre-Persyn, see Example 3) containing the cDNA fragment encoding Persyn was cleaved with 50 U *Bam*HI in the presence of 1 x reaction buffer E (Boehringer Mannheim).

Both of the above mentioned vectors were incubated (37°C, 5 minutes), separately, in the presence of 10 U T4 DNA polymerase, 1 x TE (Tris 10mM, EDTA 1 mM) and 500 µM dNTP. The enzymes were heat denatured by incubation at 75°C for 20 minutes. To each reaction 1/10 volume of 3M sodium acetate (pH 5.2) and 2 volumes 100% ethanol were added. The samples were placed at -20°C for 60 minutes and then centrifuged at 14,000 rpm for 20 minutes and the supernatant aspirated. 500 µl of ethanol (70%) was added to each tube. The tubes were centrifuged at 15,000 rpm for 2 minutes and the supernatant aspirated. The pellet was air dried for approximately 10 minutes and resuspended in 40 µl of TE (10 mM Tris, 1 mM EDTA).

Both of the above mentioned vectors were incubated (37°C, 60 minutes), separately, in the presence of 50 U (Weiss units) *Eco* RI and 1 x reaction buffer H (Boehringer Mannheim).

To the reaction containing pIRES-EGFP, 1/10 volume of 3M sodium acetate (pH 5.2) and 2 volumes 100% ethanol were added. The samples were placed at -20°C for 60 minutes and then centrifuged at 14,000 rpm for 20 minutes and the supernatant aspirated. 500 µl of ethanol (70%) was added to each tube. The tubes were centrifuged at 15,000 rpm for 2 minutes and the supernatant aspirated. The pellet was air dried for approximately 10 minutes and resuspended in 16 µl of TE (10mM Tris, 1 mM EDTA). This sample was incubated (30°C, 30 minutes), separately, in the presence of 0.02 U (Weiss units) Calf intestine alkaline phosphatase [CIAP, (Gibco BRL™)], 1 x dephosphorylation buffer and 1/20 volume dilution buffer. The enzyme was heat denatured by incubation at 75°C for 20 minutes.

The remaining sample (pTRE-Persyn) was electrophoresed in a 1% ordinary agarose gel. A DNA fragment, corresponding to the cDNA fragment encoding Persyn, was isolated from the agarose using QIAquick (QIAgen™) in exact accordance with the manufacturer's instructions.

The cDNA fragment encoding Persyn was ligated to the linearised pIRES-EGFP vector in a molar end ratio of 3:1 (insert:vector), by incubation (16°C, 18 hours) in the presence of 10 U T4 DNA ligase and 1 x ligation buffer (Promega).

Ligated DNAs were transformed into a Top10F' (Invitrogen®) bacterial host and recombinant DNAs identified by the presence of 3 DNA fragments (5 kb, ≈360 bp and ≈232 bp) upon electrophoresis in a 2% ordinary agarose gel, subsequent to cleavage in the presence of restriction enzymes *Eco* RI and *Mlu* NI.

### Example 5 : Persyn expression in skin

### MATERIALS AND METHODS

### Molecular biology techniques

RNA extraction, preparation of hybridisation probes, *in situ* and Northern hybridisations were performed as described (31, 38).

### Western blotting/ECL detection of persyn protein

Strips of skin from the back of mice were ground in liquid nitrogen, homogenised in SDS-PAGE loading buffer (39) and incubated for 5 min in a boiling water bath. Total protein concentrations were measured by the dotMETRIC assay (Geno Technology). 15 µg of total protein were used for SDS-PAGE (39). Electroblotting on Hybond-PVDF membranes was performed in TRIS/glycine/methanol buffer as recommended by the membrane supplier (Amersham). After washing with PBS, the membrane was blocked for 1 hour at room temperature in 4% skimmed milk/PBS/0.05% Tween-20. The same buffer was used for incubations with primary (SK23, 1:500) and secondary (HRP-conjugated goat anti-rabbit Ig from Amersham, 1:2000) antibodies and washes. The primary SK23 antibody was made (by Eurogentech, Belgium) by immunizing rabbits with the peptide consisting of the C-terminal 15 amino acids of mouse persyn. The final two washes were in PBS/0.1% Tween-20. ECL detection was carried out as recommended by the Amersham protocol.

### Immunohistochemistry

15 µm cryosections were fixed in acetone at -20°C for 10 minutes and were dried and blocked with 5% goat serum in TBT (20 mM TrisHCl pH7.5; 150mM NaCl; 0.1% Triton X-100) followed by incubation with the SK23 antibody (1:50) at 4°C for 16 hours in 1% goat serum/TBT. Sections were washed with 1 goat serum/TBT and processed as recommended by suppliers of secondary HRP-conjucated antibody (Sigma).

### Results and discussion

A specific polyclonal antibody (SK23) against a synthetic C-terminal peptide of mouse persyn was used for Western blot analysis of proteins extracted from different mouse tissues. This antibody detected in a single band of approximately 16kDa in samples of peripheral sensory ganglia and central nervous system (40). Adult skin was the only non-neuronal tissue in which substantial levels of persyn protein were detected. In addition to the main 16 kDa band, minor bands with slower mobilities, which probably represent aggregation products, could be seen in some skin samples. The bands in skin and neural samples disappeared when the antibodies were pre-incubated with excess of either the C-terminal peptide or recombinant GST-fused persyn protein.

Because no *persyn* mRNA has been previously detected in mouse embryonic skin we used Northern and Western blotting to ascertain the developmental time course of persyn expression in skin. Our results show that the expression of persyn mRNA and protein in skin begins on postnatal day 5 and is sustained into the adult. Similar levels of persyn protein were found in samples of total protein extracted from hairy and glaborous skin, suggesting that persyn expression is not correlated with hair growth. Both *in situ* hybridisation (Fig. 10a) and immunohistochemistry (Figs. 10b, c) demonstrated that persyn expression is restricted to stratum granulosum of the epidermis.

The physiological role of persyn and other synucleins is not clear. Recently, we showed that persyn plays a role in regulating the integrity of the neurofilament network in cultured sensory neurons (40). Our demonstration of a developmentally regulated pattern of persyn expression in the stratum granulosum raises the possibility of a parallel function for persyn in the skin. Substantial changes in the cytoskeleton, especially in the cytokeratin network, take place in the final stages of cell differentiation in the epidermis, and it is possible that persyn plays a role in rearranging the cytokeratin network in these cells. Interestingly, persyn/BCSG1 expression has been detected in some epithelial cancers, and the level of persyn expression in malignant breast tumours is correlated directly with tumour stage (41, 42). The progression from benign to highly malignant, infiltrating breast tumours is also often accompanied by structural rearrangements of cytokeratin and other intermediate filament networks (43, 44).

It has been proposed that α-synuclein functions as a molecular chaperone in the formation of Aβ-amyloid deposits and Lewy bodies in the CNS of patients with neurodegenerative diseases (7, 25). However, it is possible that in certain situations synucleins themselves produce extra- or intracellular depositions because they are able to form homopolymers and amyloid-like fibrils *in vitro* (8,9). We have shown that recombinant persyn also aggregates in solution and binds Aβ-peptides with very similar binding characteristics to that of α-synuclein. Abnormal protein and/or peptide depositions are observed not only in neurodegenerative diseases but in several pathological conditions involving other tissues. Such depositions, including depositions of Aβ-amyloid, are also observed in ageing and diseased skin (45-47), and persyn could be a candidate factor that triggers the aggregation and deposition of other macromolecules in the skin.

Taken together, our findings suggest that persyn participates in the development of the epidermis and might be involved in the pathogenesis of skin disorders. Thus, in addition to neurodegenerative and neoplastic disorders, it will be important to search for abnormalities of persyn structure and expression in skin diseases.

### Example 6

### Overexpression of Persyn in neuroblastoma cells and immunocytochemistry

### MATERIALS AND METHODS

Neuro2a (neuroblastoma) cells were grown on microcope coverslips, within 12 well tissue culture plates. 8 x 104 neuro2a cells were transiently transfected with 1.4 - 2 µg DNA (pIRES-EGFP-Persyn), in the presence of TransFastTm (Promega) at charge ratios of 1:1 and 2:1, in accordance with manufacturers instructions. Cells were incubated in the presence of 1 ml serum-free medium (Minimal essential medium, 2 mM glutamine and 1 % non-essential amino acids) for 1 hour, then overlayed with 1 ml complete medium (Minimal essential medium, 2 mM glutamine, 1 % non-essential amino acids and 10 % fetal bovine serum) and returned to the incubator for 48 hours.

### Immunocytochemistry

All medium was aspirated from wells and the cells washed with 1 ml of 1 x PBS (twice) and the saline aspirated after each wash. The cells were then immersed in 4 % paraformaldehyde and incubated at room temperature for 30 minutes. The cells were then washed with 1 ml of 1 x PBS (twice) and the saline aspirated after each wash. Cells were then overlaid with 10 µl of the antibody NE14 (Sigma, [Titre 1:40, in accordance with manufacturers recommendation]) to NF-H. The cells were then incubated, in the presence of NE14, overnight at 4°C. Cells were subsequently washed for 1-2 hours with 1 ml of 1 x PBS, the saline aspirated and replaced three times during this period. The cells were then overlaid with 10 µl (10 µg/ml) Texas Red-X (Molecular Probes) and then incubated in the dark for a period in excess of 2 hours. Cells were subsequently washed for 1-2 hours with 1 ml of 1 x PBS, the saline was aspirated and replaced three times during this period. Coverslips were then mounted on to slides using mounting medium (9:1, Glycerol: 1 x PBS [25 mg/ml DABCO]).

### Results: Overexpression of Persyn in neuroblastoma cells

To investigate the effect of overexpression of Persyn protein in neuroblastoma cells, we transiently transfected neuro2a cells with a Persyn expression/reporter plasmid. Cells positive for the expression of Persyn protein express enhanced green fluorescent protein (EGFP [Clontech]) in concert (Fig 11A & legend). A dramatic decrease in neurofilament protein NF-H was observed in neurons overexpressing persyn, compared with cells in the same field of view which had not been transfected with pIRES-EGFP-Persyn (Fig 11B,C). The marked decrease in staining was observed only in cells transfected with the Persyn expression plasmid (pIRES-EGFP-Persyn) (Fig 11C & legend). This example illustrates again that Persyn is, at least in part, responsible for the induction of changes in the pre-existing neurofilament network. Furthermore, it demonstrates that the biological activity of Persyn can be reproduced efficiently *in vitro,* within a cell based assay system, and supports the experiments reported in Example 2.

Modifications and improvements may be incorporated without departing from the scope of the invention.

### References

1. Maroteaux, L., Campanelli, J.T. & Scheller, R.H. J. Neurosci. 8, 2804-2815 (1988).
2. Maroteaux, L. & Scheller, R.H. Mol. Brain Res. 11, 335-343 (1991).
3. Uéda, K., Fukushima, H., Masliah, E., Xia, Y., Iwai, A., Yoshimoto, M., Otero, D.A.C., Kondo, J., Ihara, Y. & Saitoh, T. Proc. Natl. Acad. Sci. USA 90, 11282-11286 (1993).
4. Jakes, R., Spillantini, M.G. & Goedert, M. FEBS Lett. 345, 27-32 (1994).
5. Iwai, A., Masliah, E., Yoshimoto, M., Ge, N., Flanagan, L., de Silva, H.A.R., Kittel, A. & Saitoh, T. Neuron 14, 467-475 (1995).
6. George, J.M., Jin, H., Woods, W.S. & Clayton, D.F. Neuron 15, 361-372 (1995).
7. Yoshimoto, M., Iwai, A., Kang, D., Otero, D.A.C., Xia, Y. & Saitoh, T.9.-9. Proc. Natl. Acad. Sci. 92, 9141-9145 (1995).
8. Iwai, A., Yoshimoto, M., Masliah, E. & Saitoh, T. Biochemistry 34, 10139-10145 (1995).
9. Jensen, P.H., Hojrup, P., Hager, H., Nielsen, M.S., Jacobsen, L., Olesen, O.F., Gliemann, J. & Jakes, R. Biochem. J. 323, 539-546 (1997).
10. Polymeropoulos, M.H., et al. Science 276, 2045-2047 (1997).
11. Selkoe, D.J. Neuron 6, 487-498 (1991).
12. Kelly, J.W. Curr. Opin. Struct. Biol. 6, 11-17 (1996).
13. Wisniewski, T. & Frangione, B. Neurosci. Lett. 135, 235-238 (1992).
14. Ma, J., Yee, A., Brewer, H.B., Jr., Das., S. & Potter, H. Nature 372, 92-94 (1994).
15. Akopian, A.N. & Wood, J.N. J. Biol. Chem. 270, 21264-21270 (1995).
16. Weinreb, P.H., Zhen, W., Poon, A.W., Conway, K.A. & Lansbury, P.T.J. Biochemistry 35, 13709-13715 (1996).
17. Gilbert, D.S. & Newby, B.J. Nature 256, 586-589 (1975).
18. Wang, S., Hamberger, A., Yang, Q. & Haglid, K.G. J. Neurochem. 62, 739-748 (1994).
19. Banik, N.L., Matzelle, D.C., Gantt-Wilford, G., Osborne, A. & Hogan, E.L. Brain Res. 752, 301-306 (1997).
20. Banik, N.L., Matzelle, D.C., Terry, E. & Hogan, E.L. Brain Res. 748, 205-210 (1997).
21. Lee, K.S., Frank, S., Vanderklish, P., Arai, A. & Lynch, G. Proc. Natl. Acad. Sci. U S A 88, 7233-7237 (1991).
22. Krieglstein, J. Clin. Neurosci. 4, 184-193 (1997).
23. Wong, P.C. & Cleveland, D.W. J. Cell Biol. 111, 1987-2003 (1990).
24. Goldman, R.D., Khuon, S., Chou, Y.H., Opal, P. & Steinert, P.M. J. Cell Biol. 134, 971-983 (1996).
25. Goedert, M. Familial Parkinson's disease. Nature 388, 232-233 (1997).
26. Dickson, D.W., Wertkin, A., Kress, Y., Ksiezak Reding, H. & Yen, S.H. Lab. Invest. 63, 87-99 (1990).
27. Migheli, A., Attanasio, A., Pezzulo, T., Gullotta, F., Giordana, M.T. & Schiffer, D. Neuropathol. Appl. Neurobiol. 18, 3-11 (1992).
28. Lowe, J., Fergusson, J., Kenward, N., Laszlo, L., Landon, M., Farquhar, C., Brown, J., Hope, J. & Mayer, R.J. J. Pathol. 168, 169-177 (1992).
29. Ironside, J.W., McCardle, L., Hayward, P.A. & Bell, J.E. Neuropathol. Appl. Neurobiol. 19, 134-140 (1993).
30. Baka, I.D. & Buchman, V.L. Anal. Biochem. 237, 155-157 (1996).
31. Buchman, V.L., Ninkina, N.N., Bogdanov, Y.D., Bortvin, A.L., Akopian, H.N., Kiselev, S.L., Krylova, O.Y., Anokhin, K.V. & Georgiev, G.P. Nucleic Acids Res. 20, 5579-5585 (1992).
32. Harlow, E. & Lane, D. Antibodies: A Laboratory Manual. Cold Spring Harbor Laboratory Press, New York. (1988).
33. Ninkina, N., Grashchuck, M., Buchman, V.L. & Davies, A.M. J. Biol. Chem. 272, 13019-13025 (1997).
34. Cotman, C.W. & Taylor, D. J. Cell Biol. 55, 696-711 (1972).
35. Jones, D.H. & Matus, A.I. Biochim. Biophys. Acta 356, 276-287 (1974).
36. Buchman, V.L. & Davies, A.M. Development 118, 989-1001 (1993).
37. Ninkina, N., Adu, J., Fischer, A., Pinon, L.G., Buchman, V.L. & Davies, A.M. EMBO J. 15, 6385-6393 (1996).
38. Buchman, V.L., Sporn, M., and Davies, A.M., Development 120, 1621-1629 (1994).
39. Laemmli, E.K., Nature 227, 680-685 (1970).
40. Buchman, V.L., Adu, J., Pinõn, L.G.P., Ninkina, N.N., and Davies, A.M., Nat. Neurosci. 1, 101-103 (1998).
41. Ji, H., Liu, Y.E., Jia, T., Wang, M., Liu, J., Xiao, G., Joseph, B.K., Rosen, C., and Shi, Y.E., Cancer Res. 57, 759-764 (1997).
42. Ninkina, N., Alimova-Kost, M.V., Paterson, J.W.E., Delaney, L., Cohen, B.B., Imreh, S., Cnuchev, N.V., Davies, A.M., and Buchman, V.L., Hum.Mol.Genetics, Vol. 7, No. 9, 1417-1424 (1998).
43. Hendrix, M.J., Seftor, E.A., Chu, Y.W., Trevor, K.T., and Seftor, R.E., Metastasis Rev. 15, 507-525 (1996).
44. Hendrix, M.J., Seftor, E.A., Seftor, R.E., and Trevor, K.T., Am. J. Pathol. 150, 483-495 (1997).
45. Joachim, C.L., Mori, H., and Selkoe, D.J., Nature 341, 226-230 (1989).
46. Soininen, H., Syrjanen, S., Heinonen, O., Neittaanmaki, H., Miettinen, R., Paljarvi, L., Syrjanen, K., Beyreuther, K., and Riekkinen, P., Lancet 339, 245 (1992).
47. Wen, G.Y., Wisniewski, H.M., Blondal, H.., Benedikz, E., Frey, H., Pirttila, T., Rudelli, R. and Kim, K.S., Acta Neuropathol. (Berl) 88, 201-206 (1994).

### Annex to the description

SEQUENCE LISTING

<110> Neuropa Limited
      University Court of the University of st Andrews

<120> SYNCLEIN-BASED ASSAY AND NOVEL SYNCLEIN PROTEIN

<130> P20704-

<140>
<141>

<150> UK 9719879.0
<151> 1997-09-19

<160> 16

<170> PatentIn Ver. 2.0

<210> 1
<211> 720
<212> DNA
<213> Homo sapiens

<220>
<221> CDS
<222> (49)..(432)

<400> 1
cctgcgagct ccgtcctgcc tgcagcagca caaccctgca cacccacc atg gat gtc   57
                                                     Met Asp Val
                                                       1

ttc aag aag ggc ttc tcc atc gcc aag gag ggc gtg gtg ggt gcg gtg   105
Phe Lys Lys Gly Phe Ser Ile Ala Lys Glu Gly Val Val Gly Ala Val
      5                  10                  15

gaa aag acc aag cag ggg gtg acg gaa gca gct gag aag acc aag gag   153
Glu Lys Thr Lys Gln Gly Val Thr Glu Ala Ala Glu Lys Thr Lys Glu
 20                  25                  30                  35

ggg gtc atg tat gtg gga gcc aag acc aag gag aat gtt gta cag agc   201
Gly Val Met Tyr Val Gly Ala Lys Thr Lys Glu Asn Val Val Gln Ser
                 40                  45                  50

gtg acc tca gtg gcc gag aag acc aag gag cag gcc aac gcg gtg agc   249
Val Thr Ser Val Ala Glu Lys Thr Lys Glu Gln Ala Asn Ala Val Ser
             55                  60                  65

gag gct gtg gtg agc agc gtc aac act gtg gcc acc aag acc gtg gag     297
Glu Ala Val Val Ser Ser Val Asn Thr Val Ala Thr Lys Thr Val Glu
        70                  75                  80

gag gcg gag aac atc gcg gtc acc tcc ggg gtg gtg cgc aag gag gac     345
Glu Ala Glu Asn Ile Ala Val Thr Ser Gly Val Val Arg Lys Glu Asp
    85                  90                  95

ttg agg cca tct gcc ccc caa cag gag ggt gtg gca tcc aaa gag aaa     393
Leu Arg Pro Ser Ala Pro Gln Gln Glu Gly Val Ala Ser Lys Glu Lys
100                 105                 110                 115

gag gaa gtg gca gag gag gcc cag agt ggg gga gac tag agggctacag     442
Glu Glu Val Ala Glu Glu Ala Gln Ser Gly Gly Asp
                120                 125

gccagcgtgg atgacctgaa gagcgctcct ctgccttgga caccatcccc tcctagcaca 502

aggagtgccc gccttgagtg acatgcggct gcccacgctc ctgccctcgt cttcctggcc 562

acccttggcc tgtccacctg tgctgctgca ccaacctcac tgccctccct cggccccacc 622

caccctctgg tccttctgac cccacttatg ctgctgtgaa tttttttttt aaatgattcc 682

aaataaaact tgagcccact cctaaaaaaa aaaaaaaa                          720


<210> 2
<211> 127
<212> PRT
<213> Homo sapiens

<400> 2
Met Asp Val Phe Lys Lys Gly Phe Ser Ile Ala Lys Glu Gly Val Val
 1               5                  10                  15

Gly Ala Val Glu Lys Thr Lys Gln Gly Val Thr Glu Ala Ala Glu Lys
            20                  25                  30

Thr Lys Glu Gly Val Met Tyr Val Gly Ala Lys Thr Lys Glu Asn Val
        35                  40                  45

Val Gln Ser Val Thr Ser Val Ala Glu Lys Thr Lys Glu Gln Ala Asn
    50                  55                  60

Ala Val Ser Glu Ala Val Val Ser Ser Val Asn Thr Val Ala Thr Lys
65                  70                  75                  80

Thr Val Glu Glu Ala Glu Asn Ile Ala Val Thr Ser Gly Val Val Arg
                85                  90                  95

Lys Glu Asp Leu Arg Pro Ser Ala Pro Gln Gln Glu Gly Val Ala Ser
            100                 105                 110

Lys Glu Lys Glu Glu Val Ala Glu Glu Ala Gln Ser Gly Gly Asp
        115                 120                 125

<210> 3
<211> 727
<212> DNA
<213> Mus musculus

<220>
<221> CDS
<222> (69)..(440)

<400> 3
cagagacagc ggctgcgcac gctccagtcc atagcttgca gcagccaggt tccatccttg 60

caaacacc atg gac gtc ttc aag aaa ggc ttc tcc att gcc aag gaa ggt  110
         Met Asp Val Phe Lys Lys Gly Phe Ser Ile Ala Lys Glu Gly
           1               5                  10

gtt gtg ggt gct gta gaa aag acc aag cag gga gta acg gag gca gct   158
Val Val Gly Ala Val Glu Lys Thr Lys Gln Gly Val Thr Glu Ala Ala
 15                  20                  25                  30

gag aag acc aag gag ggg gtt atg tat gtg ggc acc aaa acc aag gag   206
Glu Lys Thr Lys Glu Gly Val Met Tyr Val Gly Thr Lys Thr Lys Glu
                35                  40                  45

aac gtg gta caa agt gtc acc tca gtg gct gag aag acc aag gag cag   254
Asn Val Val Gln Ser Val Thr Ser Val Ala Glu Lys Thr Lys Glu Gln
            50                  55                  60

gcc aat gcc gtg agt gaa gct gtg gtc agc agc gtc aac aca gtg gcc   302
Ala Asn Ala Val Ser Glu Ala Val Val Ser Ser Val Asn Thr Val Ala
        65                  70                  75

aac aag acc gtg gag gag gct gaa aac atc gtg gtc acc acc ggg gtg   350
Asn Lys Thr Val Glu Glu Ala Glu Asn Ile Val Val Thr Thr Gly Val
    80                  85                  90

gtg cgc aag gag gac ttg gaa ccc cct gca cag gac caa gaa gcc aaa   398

Val Arg Lys Glu Asp Leu Glu Pro Pro Ala Gln Asp Gln Glu Ala Lys
95                  100                 105                 110

gag caa gag gag aat gaa gag gcc aag agt gga gaa gac tag             440
Glu Gln Glu Glu Asn Glu Glu Ala Lys Ser Gly Glu Asp
                115                 120

aaggctgcca gccaaccaca gaggctcaag ggacacccct ctgccttgga ccctgtctca 500

acctggcaca ctgaatgccc tgcctagagt gccctgcagg ttgcccaagt tctctgtccc 560

tagcccagtg ccacaagtcc acacacgcta ctgccagcgc cagcaccccc tgctggctcc 620

ttttacccta tggtcctctg atcaccaatc agatgctgct gtgatttttt tttttttaat 680

gattccaaat aaaacctgag ccccactccc caaaaaaaaa aaaaaaa                727

<210> 4
<211> 123
<212> PRT
<213> Mus musculus

<400> 4
Met Asp Val Phe Lys Lys Gly Phe Ser Ile Ala Lys Glu Gly Val Val
1               5                   10                  15

Gly Ala Val Glu Lys Thr Lys Gln Gly Val Thr Glu Ala Ala Glu Lys
            20                  25                  30

Thr Lys Glu Gly Val Met Tyr Val Gly Thr Lys Thr Lys Glu Asn Val
        35                  40                  45

Val Gln Ser Val Thr Ser Val Ala Glu Lys Thr Lys Glu Gln Ala Asn
    50                  55                  60

Ala Val Ser Glu Ala Val Val Ser Ser Val Asn Thr Val Ala Asn Lys
65                  70                  75                  80

Thr Val Glu Glu Ala Glu Asn Ile Val Val Thr Thr Gly Val Val Arg
                85                  90                  95

Lys Glu Asp Leu Glu Pro Pro Ala Gln Asp Gln Glu Ala Lys Glu Gln
            100                 105                 110

Glu Glu Asn Glu Glu Ala Lys Ser Gly Glu Asp
        115                 120

<210> 5
<211> 113
<212> PRT
<213> Torpedo californica

<400> 5
Met Asp Val Leu Lys Lys Gly Phe Ser Phe Ala Lys Glu Gly Lys Glu
1               5                   10                  15

Gly Val Met Tyr Val Gly Thr Lys Thr Lys Glu Gly Val Val Gln Ser
            20                  25                  30

Val Asn Thr Val Thr Glu Lys Thr Lys Glu Gln Ala Asn Val Val Gly
        35                  40                  45

Gly Ala Val Val Ala Gly Val Asn Thr Val Ala Ser Lys Thr Val Glu
    50                  55                  60

Gly Val Glu Asn Val Ala Ala Ala Ser Gly Val Val Lys Leu Asp Glu
65                  70                  75                  80

His Gly Arg Glu Ile Pro Ala Glu Gln Val Ala Glu Gly Lys Gln Thr
                85                  90                  95

Thr Gln Glu Pro Leu Val Glu Ala Thr Glu Ala Thr Glu Glu Thr Gly
            100                 105                 110

Lys

<210> 6
<211> 140
<212> PRT
<213> Rattus sp.

<400> 6
Met Asp Val Phe Met Lys Gly Leu Ser Lys Ala Lys Glu Gly Val Val
1               5                   10                  15

Ala Ala Ala Glu Lys Thr Lys Gln Gly Val Ala Glu Ala Ala Gly Lys
            20                  25                  30

Thr Lys Glu Gly Val Leu Tyr Val Gly Ser Lys Thr Lys Glu Gly Val
        35                  40                  45

Val His Gly Val Thr Thr Val Ala Glu Lys Thr Lys Glu Gln Val Thr

50                  55                  60

Asn Val Gly Gly Ala Val Val Thr Gly Val Thr Ala Val Ala Gln Lys
65                  70                  75                  80

Thr Val Glu Gly Ala Gly Asn Ile Ala Ala Ala Thr Gly Phe Val Lys
                85                  90                  95

Lys Asp Gln Met Gly Lys Gly Glu Glu Gly Tyr Pro Gln Glu Gly Ile
            100                 105                 110

Leu Glu Asp Met Pro Val Asp Pro Ser Ser Glu Ala Tyr Glu Met Pro
        115                 120                 125

Ser Glu Glu Gly Tyr Gln Asp Tyr Glu Pro Glu Ala
    130                 135                 140

<210> 7
<211> 149
<212> PRT
<213> Rattus sp.

<400> 7
Met Asp Val Phe Met Lys Gly Leu Ser Lys Ala Lys Glu Gly Val Val
1               5                   10                  15

Ala Ala Ala Glu Lys Thr Lys Gln Gly Val Ala Glu Ala Ala Gly Lys
            20                  25                  30

Thr Lys Glu Gly Val Leu Tyr Val Gly Ser Lys Thr Lys Glu Gly Val
        35                  40                  45

Val His Gly Val Thr Thr Val Ala Glu Lys Thr Lys Glu Gln Val Thr
    50                  55                  60

Asn Val Gly Gly Ala Val Val Thr Gly Val Thr Ala Val Ala Gln Lys
65                  70                  75                  80

Thr Val Glu Gly Ala Gly Asn Ile Ala Ala Ala Thr Gly Phe Val Lys
                85                  90                  95

Lys Asp Gln Met Gly Lys Gly Tyr Pro Met Gly Glu Cys Thr Asn His
            100                 105                 110

Pro Pro Arg Leu Ile Ala Leu Arg Val Lys Ser Arg Tyr Arg Glu His
        115                 120                 125

Ser Trp Arg Pro Arg Lys Gln Leu Ser Leu Ala Cys Val Val Met Asp
    130                 135                 140

Pro Phe Leu Pro Thr
145

<210> 8
<211> 42
<212> PRT
<213> Rattus sp.

<400> 8
Met Asp Val Phe Met Lys Gly Leu Ser Lys Ala Lys Glu Gly Val Val
  1               5                  10                  15

Ala Ala Ala Glu Lys Thr Lys Gln Gly Val Ala Glu Ala Ala Gly Lys
             20                  25                  30

Thr Lys Glu Gly Val Leu Tyr Val Gly Arg
         35                  40

<210> 9
<211> 140
<212> PRT
<213> Homo sapiens

<400> 9

<210> 10
<211> 134
<212> PRT
<213> Homo sapiens

<400> 10
Met Asp Val Phe Met Lys Gly Leu Ser Met Ala Lys Glu Gly Val Val
1               5                   10                  15

Ala Ala Ala Glu Lys Thr Lys Gln Gly Val Thr Glu Ala Ala Glu Lys
            20                  25                  30

Thr Lys Glu Gly Val Leu Tyr Val Gly Ser Lys Thr Arg Glu Gly Val
        35                  40                  45

Val Gln Gly Val Ala Ser Val Ala Glu Lys Thr Lys Glu Gln Ala Ser
    50                  55                  60

His Leu Gly Gly Ala Val Phe Ser Gly Ala Gly Asn Ile Ala Ala Ala
65                  70                  75                  80

Thr Gly Leu Val Lys Arg Glu Glu Phe Pro Thr Asp Leu Lys Pro Glu
                85                  90                  95

Glu Val Ala Gln Glu Ala Ala Glu Glu Pro Leu Ile Glu Pro Leu Met
            100                 105                 110

Glu Pro Glu Gly Glu Ser Tyr Glu Asp Pro Pro Gln Glu Glu Tyr Gln
        115                 120                 125

Glu Tyr Glu Pro Glu Ala
    130

<210> 11
<211> 133
<212> PRT
<213> Bos taurus

<400> 11
Met Asp Val Phe Met Lys Gly Leu Ser Lys Ala Lys Glu Gly Val Val
1               5                   10                  15

Ala Ala Ala Glu Lys Thr Lys Gln Gly Val Ala Glu Ala Ala Gly Lys
            20                  25                  30

Thr Lys Glu Gly Val Leu Tyr Val Gly Ser Lys Thr Lys Glu Gly Val
        35                  40                  45

Val His Gly Val Ala Thr Val Ala Glu Lys Thr Lys Glu Gln Ala Ser
    50                  55                  60

His Leu Gly Gly Ala Val Phe Ser Gly Ala Gly Asn Ile Ala Ala Ala
65                  70                  75                  80

Thr Gly Val Lys Lys Glu Glu Phe Pro Thr Asp Leu Lys Pro Glu Glu
                85                  90                  95

Val Ala Gln Glu Ala Ala Glu Glu Pro Leu Ile Glu Pro Leu Met Glu
            100                 105                 110

Pro Glu Gly Glu Ser Tyr Glu Glu Gln Pro Gln Glu Glu Tyr Gln Glu
        115                 120                 125

Tyr Glu Pro Glu Ala
    130



<210> 12
<211> 143
<212> PRT
<213> Serinus canaria

<400> 12
Met Asp Val Phe Met Lys Gly Leu Ser Lys Ala Lys Glu Gly Val Val
1               5                   10                  15

Ala Ala Ala Glu Lys Thr Lys Gln Gly Val Ala Glu Ala Ala Gly Lys
            20                  25                  30

Thr Lys Glu Gly Val Leu Tyr Val Gly Ser Arg Thr Lys Glu Gly Val
        35                  40                  45

Val His Gly Val Thr Thr Val Ala Glu Lys Thr Lys Glu Gln Val Ser
    50                  55                  60

Asn Val Gly Gly Ala Val Val Thr Gly Val Thr Ala Val Ala Gln Lys

65                  70                  75                  80

Thr Val Glu Gly Ala Gly Asn Ile Ala Ala Ala Thr Gly Leu Val Lys
                85                  90                  95

Lys Asp Gln Leu Ala Lys Gln Asn Glu Glu Gly Phe Leu Gln Glu Gly
            100                 105                 110

Met Val Asn Asn Thr Gly Ala Ala Val Asp Pro Asp Asn Glu Ala Tyr
        115                 120                 125

Glu Met Pro Pro Glu Glu Glu Tyr Gln Asp Tyr Glu Pro Glu Ala
    130                 135                 140

<210> 13
<211> 123
<212> PRT
<213> Rattus norvegicus

<400> 13
Met Asp Val Phe Lys Lys Gly Phe Ser Ile Ala Arg Glu Gly Val Val
1               5                   10                  15

Gly Ala Val Glu Lys Thr Thr Gln Gly Val Thr Glu Ala Ala Glu Lys
            20                  25                  30

Thr Lys Glu Gly Val Met Tyr Val Gly Ala Lys Thr Lys Gly Glu Arg
        35                  40                  45

Gly Thr Ser Val Thr Ser Val Ala Glu Lys Thr Lys Glu Gln Ala Asn
    50                  55                  60

Ala Val Ser Glu Ala Val Val Ser Ser Val Asn Thr Val Ala Thr Glu
65                  70                  75                  80

Thr Val Glu Glu Ala Glu Asn Ile Val Val Thr Thr Gly Val Val Arg
                85                  90                  95

Lys Glu Asp Leu Glu Pro Pro Ala Gln Asp Gln Glu Ala Lys Glu Gln
            100                 105                 110

Glu Glu Gly Glu Glu Ala Lys Ser Gly Gly Asp
        115                 120

<210> 14
<211> 27

<212> DNA
<213> Artificial Sequence

<220>
<223> Description of Artificial Sequence: synthetic
      sense oligonucleotide

<400> 14
ggaattccat ccttgcaaac accatgg                                          27


<210> 15
<211> 29
<212> DNA
<213> Artificial Sequence

<220>
<223> Description of Artificial Sequence: Synclein
      partial motif found to be identical in diferent
      species.

<400> 15
cgggatccct agtcttctcc actcttggc                                        29


<210> 16
<211> 7
<212> PRT
<213> Artificial Sequence

<220>
<223> Description of Artificial Sequence: Amino Acid
      motif characteristic of syncleins

<400> 16
Glu Lys Thr Lys Glu Gly Val
  1               5

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

TO Dr V L Buchman
University of St. Andrews
School of Biomedical Sciences
Bute Medical Building
ST. ANDREWS
Fife
KY16 9TS
NAME AND ADDRESS
OF DEPOSITOR

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the DEPOSITOR: | Accession number given by the INTERNATIONAL DEPOSITARY AUTHORITY:
E. coli K-12, B834, [?] PERS-GEX | NCTC 13049

II. SCIENTIFIC DESCRIPTION AND/OR PROPOSED TAXONOMIC DESIGNATION
The microorganism identified under I above was accompanied by:
[ ] a scientific description
[X] a proposed taxonomic designation
(Mark with a cross where applicable)

III. RECEIPT AND ACCEPTANCE
This International Depositary Authority accepts the microorganism identified under I above,
which was received by it on 18th September 1997 (date of original deposit) 1

IV. INTERNATIONAL DEPOSITARY AUTHORITY
Name: National Collection of Type Cultures | Signature(s) of person(s) having the power to represent the International Depositary Authority or of authorized official(s):
Address: Central Public Health Laboraatory
61 Colindale Avenue
LONDON, NW9 5HT | Date: 18/11/97 Barry Holmes
B Holmes
Clinical Scientist

1 Where Rule 6.4(d) applies, such date is the date on which the status of international depositary
authority was acquired; where a deposit made outside the Budapest Treaty after the acquisition
of the status of international depositary authority is converted into a deposit under the
Budapest Treaty, such date is the date on which the microorganism was received by the
international depositary authority.

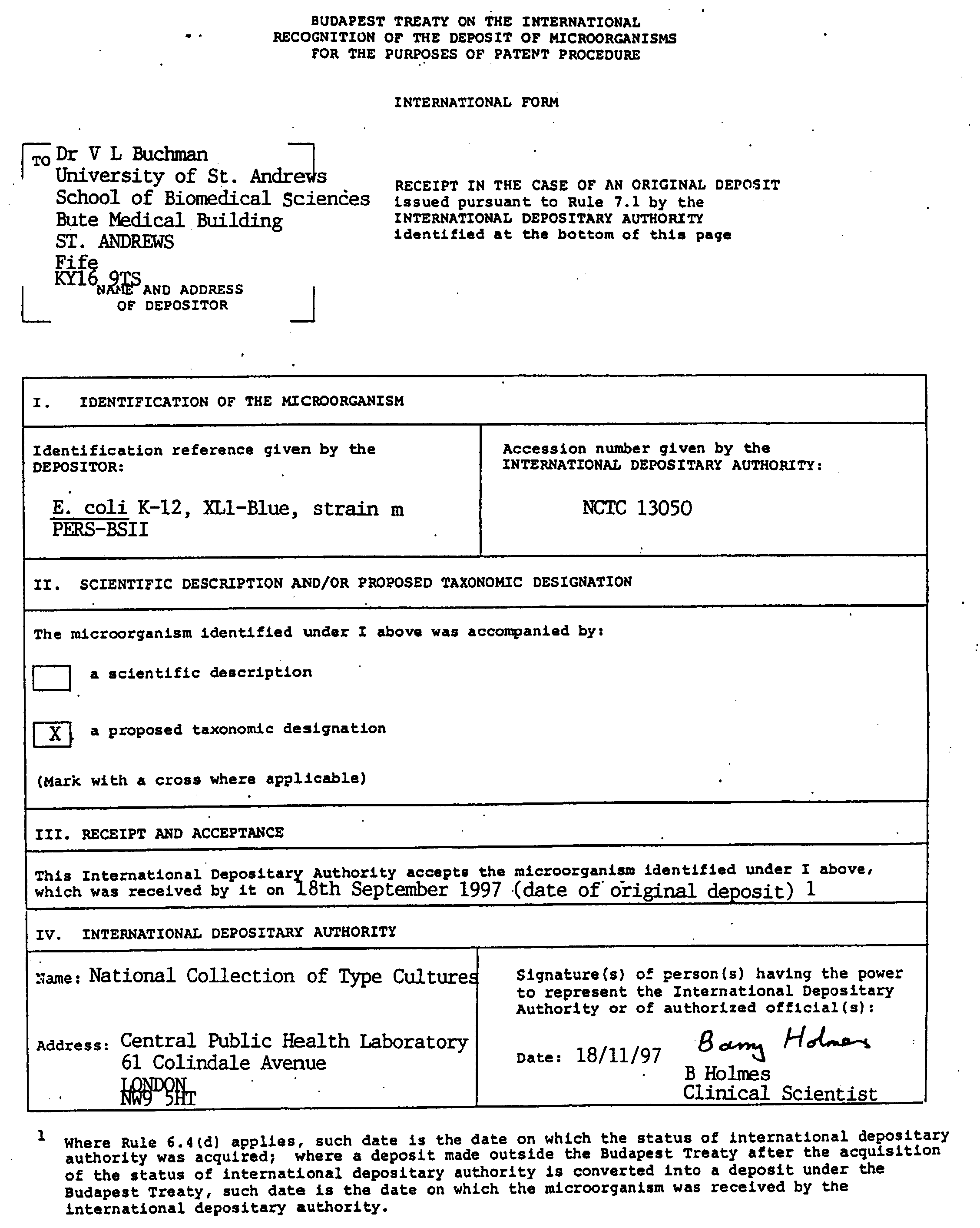
BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

TO Dr V L Buchman
University of St. Andrews
School of Biomedical Sciences
Bute Medical Building
ST. ANDREWS
Fife
KY16 9TS
NAME AND ADDRESS
OF DEPOSITOR

RECEIPT IN THE CASE OF AN ORIGINAL DEPOSIT
issued pursuant to Rule 7.1 by the
INTERNATIONAL DEPOSITARY AUTHORITY
identified at the bottom of this page

I. IDENTIFICATION OF THE MICROORGANISM
Identification reference given by the DEPOSITOR: | Accession number given by the INTERNATIONAL DEPOSITARY AUTHORITY:
E. coli K-12, XL1-Blue, strain m PERS-BSII | NCTC 13050

II. SCIENTIFIC DESCRIPTION AND/OR PROPOSED TAXONOMIC DESIGNATION
The microorganism identified under I above was accompanied by:
[ ] a scientific description
[X] a proposed taxonomic designation
(Mark with a cross where applicable)

III. RECEIPT AND ACCEPTANCE
This International Depositary Authority accepts the microorganism identified under I above, which was received by it on 18th September 1997 (date of original deposit) 1

IV. INTERNATIONAL DEPOSITARY AUTHORITY
Name: National Collection of Type Cultures | Signature(s) of person(s) having the power to represent the International Depositary Authority or of authorized official(s):
Address: Central Public Health Laboratory
61 Colindale Avenue
LONDON
NW9 5HT | Date: 18/11/97 B Holmes
Clinical Scientist

1 Where Rule 6.4(d) applies, such date is the date on which the status of international depositary authority was acquired; where a deposit made outside the Budapest Treaty after the acquisition of the status of international depositary authority is converted into a deposit under the Budapest Treaty, such date is the date on which the microorganism was received by the international depositary authority.

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

TO University of St. Andrews
School of Biomedical Sciences
Bute Medical Building
ST. ANDREWS
Fife
KY16 9TS

(Attn: Dr V. L. Buchman)
NAME AND ADDRESS OF THE PARTY
TO WHOM THE VIABILITY STATEMENT
IS ISSUED

VIABILITY STATEMENT
issued pursuant to Rule 10.2 by the
INTERNATIONAL DEPOSITARY AUTHORITY
identified on the following page

I. DEPOSITOR | II. IDENTIFICATION OF THE MICROORGANISM
Name: Dr V. L. Buchman | Accession number given by the INTERNATIONAL DEPOSITARY AUTHORITY:
Address: as above | NCTC 13049
| Date of the deposit or of the transfer:
| 18th September 1997

III. VIABILITY STATEMENT
The viability of the microorganism identified under II above was tested
on 2nd October 1997 2. On that date, the said microorganism was

[X] 3 viable

[ ] 3 no longer viable

1 Indicate the date of the original deposit or, where a new deposit or a transfer has been
made, the most recent relevant date (date of the new deposit or date of the transfer).

2 In the cases referred to in Rule 10.2(a)(ii) and (iii), refer to the most recent viability
test.

3 Mark with a cross the applicable box.

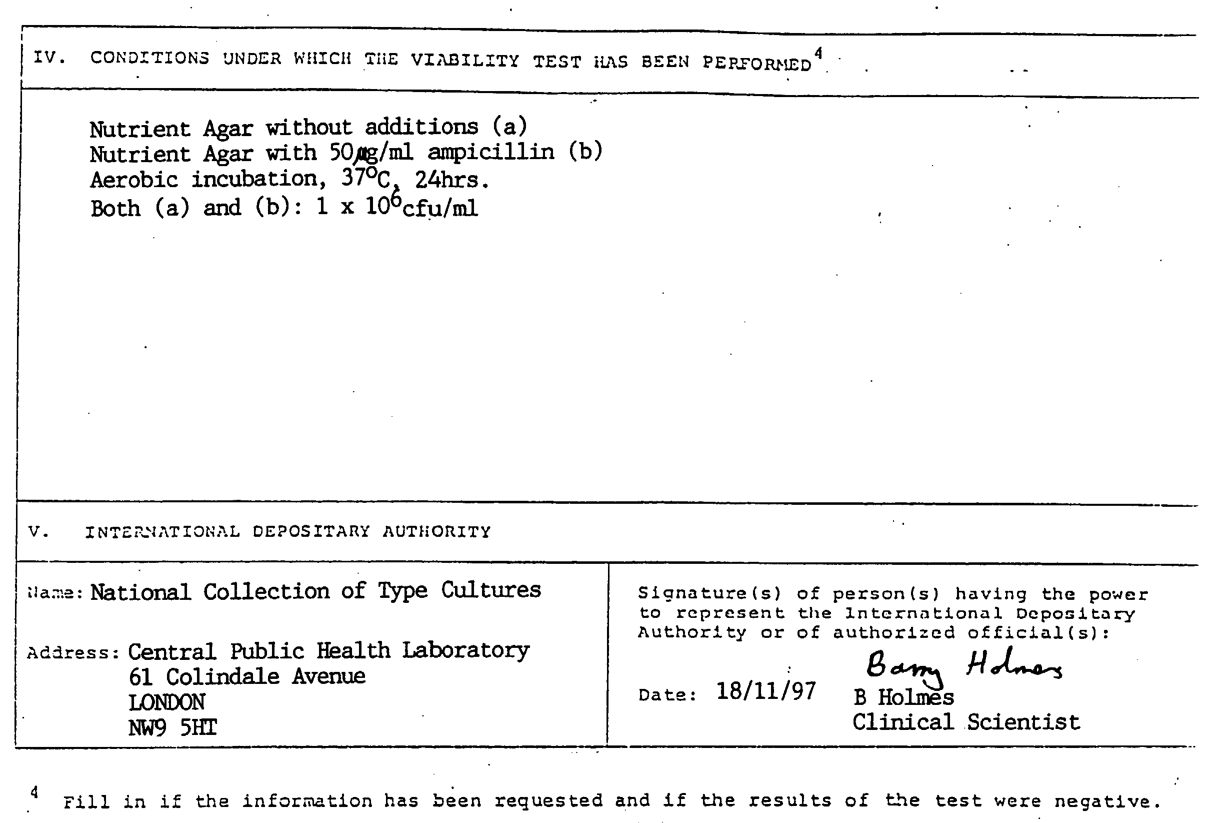
IV. CONDITIONS UNDER WHICH THE VIABILITY TEST HAS BEEN PERFORMED⁴

Nutrient Agar without additions (a)
Nutrient Agar with 50µg/ml ampicillin (b)
Aerobic incubation, 37°C, 24hrs.
Both (a) and (b): 1 x 10⁶cfu/ml

V. INTERNATIONAL DEPOSITARY AUTHORITY

Name: National Collection of Type Cultures | Signature(s) of person(s) having the power
 | to represent the International Depositary
Address: Central Public Health Laboratory | Authority or of authorized official(s):
61 Colindale Avenue |
LONDON | Date: 18/11/97 B Holmes
NW9 5HT | Clinical Scientist

⁴ Fill in if the information has been requested and if the results of the test were negative.

BUDAPEST TREATY ON THE INTERNATIONAL
RECOGNITION OF THE DEPOSIT OF MICROORGANISMS
FOR THE PURPOSES OF PATENT PROCEDURE

INTERNATIONAL FORM

TO University of St. Andrews
School of Biomedical Sciences
Bute Medical Building
ST. ANDREWS
Fife
KY16 9TS

(Attn: Dr V. L. Buchman)

NAME AND ADDRESS OF THE PARTY
TO WHOM THE VIABILITY STATEMENT
IS ISSUED

VIABILITY STATEMENT
issued pursuant to Rule 10.2 by the
INTERNATIONAL DEPOSITARY AUTHORITY
identified on the following page

I. DEPOSITOR | II. IDENTIFICATION OF THE MICROORGANISM
Name: Dr V. L. Buchman | Accession number given by the
Address: as above | INTERNATIONAL DEPOSITARY AUTHORITY:
| NCTC 13050
| Date of the deposit or of the transfer:
| 18th September 1997

III. VIABILITY STATEMENT

The viability of the microorganism identified under II above was tested
on 2nd October 1997. 2. On that date, the said microorganism was

[X] 3 viable

[ ] 3 no longer viable

1 Indicate the date of the original deposit or, where a new deposit or a transfer has been
made, the most recent relevant date (date of the new deposit or date of the transfer).

2 In the cases referred to in Rule 10.2(a)(ii) and (iii), refer to the most recent viability
test.

3 Mark with a cross the applicable box.

IV. CONDITIONS UNDER WHICH THE VIABILITY TEST HAS BEEN PERFORMED⁴

Nutrient Agar without additions (a)
Nutrient Agar with 50µg/ml ampicillin (b)
Aerobic incubation, 37°C, 24 hrs.
Both (a) and (b): 1 x 10⁶cfu/ml

V. INTERNATIONAL DEPOSITARY AUTHORITY

Name: National collection of type Cultures | Signature(s) of person(s) having the power
 | to represent the International Depositary
Address: Central Public Health Laboratory | Authority or of authorized official(s):
61 Colindale Avenue |
LONDON | Date: 18/11/97 Barry Holmes
NW9 5HT | B Holmes
 | Clinical Scientist

⁴ Fill in if the information has been requested and if the results of the test were negative.

## Claims

1. A method of diagnosis of, or determining the suspectibility to, disease states or disorders characterised by overdegradation or accumulation of synuclein-sensitive protein, said method comprising:
a) obtaining an appropriate tissue sample from a patient;
b) analysing the presence or characteristics of a synuclein from said sample; and
c) comparing the results obtained to a known standard.

2. A method as claimed in Claim 1 wherein said method comprises:
a. obtaining an appropriate tissue sample from a patient;
b. determining the *ex vivo* level of a synuclein (for example persyn) in said sample; and
c. comparing that level to a known standard.

3. A method as claimed in Claim 1 wherein said method comprises:
a. obtaining an appropriate tissue sample from a patient;
b. isolating a synuclein (for example persyn) or a polynucleotide encoding therefor, from said sample;
c. comparing said synuclein or said polynucleotide to a known standard and determining whether there are any relevant differences (for example differences in sequence or physical characteristics) therein.

4. A method as claimed in any one of Claims 1 to 3 wherein said synuclein-sensitive proteins are intermediate filament proteins.

5. A method as claimed in Claim 4 wherein said synuclein-sensitive proteins are neurofilament proteins or cytokeratin proteins.

6. A method as claimed in any one of Claims 1 to 5 wherein the synuclein of step b is human persyn, (or isoforms, modifications or functional equivalents thereof), mouse α-synuclein, human α-synuclein or human β-synuclein.

7. A method as claimed in Claim 6 wherein the synuclein of step b is human persyn.

8. A method as claimed in any one of Claims 1 to 7 wherein the known standard of step c is human persyn or mouse persyn (or isoforms, modifications or functional equivalents thereof), human α-synuclein or human β-synuclein.

9. A method as claimed in Claim 8 wherein the synuclein of step c is human persyn or mouse persyn.

10. A method as claimed in any one of Claims 1 to 9 wherein the known standard of step c is a synuclein expressed from a stable cell line under the control of an inducible promoter.

11. A method as claimed in Claim 10 wherein the synuclein is expressed from a stable cell line under the control of a tetracycline or tetracycline analogue inducible promoter.

12. A method as claimed in any one of Claims 1 to 11 for screening, diagnosing or monitoring cancer, neurodegenerative disorders or skin disorders.

13. A method as claimed in any one of Claims 1 to 12 for the diagnosis of breast cancer or skin cancer.

14. A method as claimed in any one of Claims 1 to 12 for the diagnosis of neurodegenerative disorders.

15. A method of combatting intermediate filament damage in cells due to abnormal intracellular levels of a synuclein, said method comprising identifying the cells affected and modifying the biologically effective amount of said synuclein therein.

16. A method as claimed in Claim 15 wherein said cells having intermediate filament damage are identified by a method as claimed in any one of Claims 1 to 14.

17. A method as claimed in either one of Claims 15 and 16 wherein said biologically effective amount of said synuclein is reduced due to the presence of anti-sense RNA.

18. A method as claimed in either one of Claims 15 and 16 wherein said biologically effective amount of said synuclein is reduced due to homologous recombination of the gene(s) therefor with a defective version thereof.

19. A method of screening for a therapeutic agent able to combat the biological activity of a synuclein, said method comprising:
a. obtaining a system capable of demonstrating the biological activity of the synuclein;
b. exposing said system to a putative therapeutic agent; and
c. determining whether said agent affects the biological activity of the synuclein.

20. A method as claimed in Claim 19 wherein the system of step a is a primary cell culture or cell line transfected with an expression system for the synuclein.

21. A method as claimed in Claim 20 wherein the synuclein expression system is a synuclein pIRES-EGFP vector.

22. A method as claimed in Claim 19 wherein the system of step a is a primary cell culture or cell line micro-injected with the synuclein.

23. A method as claimed in any one of Claims 20 to 22 wherein the system of step a is a keratinocyte, a neural progenitor, a neuroblast or a neural-like cell line.

24. A method as claimed in any one of Claims 19 to 23 wherein said synuclein is persyn.

25. A method as claimed in any one of Claims 19 to 24 wherein the synuclein is expressed from a stable cell line under the control of an inducible promoter.

26. A method as claimed in Claim 25 wherein the synuclein is expressed from a stable cell line under the control of a tetracycline or tetracycline analogue inducible promoter.

27. Persyn having an amino acid sequence substantially as set out in SEQ ID No 2 or in SEQ ID No 4.

28. Use of persyn as claimed in Claim 27 in a method of diagnosis of cancers, neurodegenerative disorder or skin disorders, which condition involves alteration of the intracellular intermediate filament structure in the tissue affected.

29. A polynucleotide comprising a nucleotide sequence substantially as set out in SEQ ID No 1 or SEQ ID No 3.

30. A polynucleotide comprising a nucleotide sequence encoding persyn.

31. A recombinant genetic expression system comprising a polynucleotide as claimed in either one of Claims 29 and 30 and able to express persyn.

32. A vector comprising a polynucleotide as claimed in either one of Claims 29 and 30.

33. A vector comprising an expression system as claimed in Claim 31.

34. The vector hPERS-GEX deposited at NCTC under Accession No 13049.

35. The vector mPERS-BSII deposited at NCTC under Accession No 13050.

36. A host cell transformed with a vector as claimed in any one of Claims 32 to 35.

37. A transgenic mammalian animal having a recombinant expression system as claimed in Claim 31 stably integrated into its genome.

38. Persyn expressed from a polynucleotide as claimed in either one of Claims 29 and 30.

39. Use of persyn as claimed in Claim 38 in a method of diagnosis of cancers, neurodegenerative disorders or skin disorders, which condition involves alteration of the intracellular intermediate filament structure in the tissues affected.
